(19) Europäisches Patentamt
European Patent Office
Office européen des brevets

(11) **EP 1 632 371 B1**

(12) **EUROPEAN PATENT SPECIFICATION**

(45) Date of publication and mention
of the grant of the patent:
**06.06.2007 Bulletin 2007/23**

(51) Int Cl.:
***B60H 1/00*** *(2006.01)*     ***B60H 3/00*** *(2006.01)*
***A61B 5/00*** *(2006.01)*

(21) Application number: **05002800.0**

(22) Date of filing: **10.02.2005**

(54) **Air conditioner for vehicle cabin**

Klimaanlage für Kraftfahrzeuginnenraum

Climatisation pour habitale

(84) Designated Contracting States:
**DE**

(30) Priority: **06.09.2004 JP 2004257835**

(43) Date of publication of application:
**08.03.2006 Bulletin 2006/10**

(73) Proprietor: **Hitachi Ltd.**
**Tokyo 100-8280 (JP)**

(72) Inventors:
- **Kono, Miyuki**
  **c/o Hitachi, Ltd. Int. Prop. Group**
  **Chiyoda-ku**
  **Tokyo 100-8220 (JP)**
- **Kashimura, Yuichi**
  **c/o Hitachi Ltd Int. Prop. Group**
  **Chiyoda-ku**
  **Tokyo 100-8220 (JP)**
- **Matsumura, Takafumi**
  **c/o Hitachi Ltd I. Prop. Group**
  **Chiyoda-ku**
  **Tokyo 100-8220 (JP)**
- **Miyatake, Takafumi**
  **c/o Hitachi Ltd In. Prop. Group**
  **Chiyoda-ku**
  **Tokyo 100-8220 (JP)**
- **Nagasaka, Akio**
  **c/o Hitachi, Ltd Int. Prop. Group**
  **Chiyoda-ku**
  **Tokyo 100-8220 (JP)**
- **Masuzawa, Hiroshi**
  **c/o Hitachi Ltd Int. Prop. Group**
  **Chiyoda-ku**
  **Tokyo 100-8220 (JP)**
- **Miura, Naoto**
  **c/o Hitachi, Ltd. Int. Prop. Group**
  **Chiyoda-ku**
  **Tokyo 100-8220 (JP)**

(74) Representative: **Beetz & Partner**
**Steinsdorfstrasse 10**
**80538 München (DE)**

(56) References cited:
**EP-A- 1 354 738**     **DE-A1- 19 955 082**
**DE-C1- 10 233 727**

- **PATENT ABSTRACTS OF JAPAN vol. 2000, no. 16, 8 May 2001 (2001-05-08) & JP 2001 022919 A (MINOLTA CO LTD), 26 January 2001 (2001-01-26)**

**Description**

**FIELD OF THE INVENTION**

The present invention relates to an air conditioner in the cabin of a vehicle such as automobile and train.

**BACKGROUND OF THE INVENTION**

A shortage of oxygen in a cabin while an occupant is driving a vehicle is thought to be one cause of reducing the degree of concentration of the occupant and making the occupant get sleepy. Further, a shortage of oxygen during driving in highlands causes high-altitude thickness and, in particular, driving the vehicle in such a way as to increase altitude rapidly is extremely dangerous. For this reason, it is thought that the need for a technology of preventing a high-altitude sickness is great.

As a method for making up for a shortage of oxygen in the vehicle cabin is proposed a technology for separating and removing nitrogen in the atmosphere to produce air enriched with oxygen, as disclosed in Japanese Patent Laid-Open No. S63(1988)-43812. Further, as disclosed in Japanese Patent Laid-Open No. H6 (1994)-92140, there is proposed a technology for quantifying the degree of fatigue of a vehicle driver by the number of displacements of a suspension and supplying air enriched with oxygen to the vehicle cabin when the number of displacements of a suspension is larger than a predetermined number and oxygen concentration in the vehicle cabin is lower than a predetermined concentration.

EP 1 354 738 A1, which is considered as the closest prior art, discloses a device to prevent that polluting agents, such as toxic powders and gas may enter the driver's cabin of a motor vehicle and damage the passengers. To avoid that too long expositions to polluting agents and consequently too long periods of breathing of the internal air may cause an increase in CO2 percentage and then damages to the passengers, a CO2 absorber is put in the air circuit in order to absorb CO2 produced by passengers' breathing. An O2 sensor is put in the cabin of the motor vehicle to activate the intake of O2 into the vehicle from an external small cylinder in order to maintain the right percentage of O2 in the cabin.

**SUMAMRY OF THE INVENTION**

However, the method disclosed in Japanese Patent Laid-Open No. S63(1988)-43812 can not find the states where individual occupants are short of oxygen, so that it is impossible to take appropriate action responsive to the state.

On the other hand, according to the technology disclosed in Japanese Patent Laid-Open No. H6 (1994)-92140, the degree of fatigue quantified on the basis of the number of displacements of a suspension is an estimated value and the extent to which the occupant is actually short of oxygen is not actually measured.

Further, when an occupant drives a vehicle in highlands, the occupant is in danger of developing high-altitude sickness but with the current state of the art, countermeasures against the high-altitude sickness are hardly undertaken in the vehicle.

The present invention is to provide an apparatus that conditions air in a vehicle cabin on the basis of results obtained by detecting conditions typified by a shortage of oxygen of individual occupants in the vehicle cabin and by detecting a change in the amount of constituent component of air, which is typified by oxygen, to eliminate the deterioration of air environment typified by a shortage of oxygen. Further, the invention is to provide also an apparatus for preventing an occupant from developing high-altitude sickness when the occupant drives a vehicle in highlands.

The invention provides a control unit according to claims 1 and 7, respectively, and a method according to claim 10.

The present invention is an air conditioning system characterized by including: an oxygen saturation detecting sensor for measuring oxygen saturation in the blood of a driver or a fellow passenger; an air conditioner for supplying air to a vehicle cabin; and a control unit for controlling the oxygen concentration of air supplied by the air conditioner according to the oxygen saturation measured by the oxygen saturation detecting sensor.

According to the invention, it is possible to detect the state where the vehicle cabin is or the individual occupants are short of oxygen and to eliminate a shortage of oxygen. Further, it is also possible to prevent the occupant from developing high-altitude sickness when the occupant drives the vehicle in highlands.

**BRIEF DESCRIPTION OF THE DRAWINGS**

FIGS. 1A and 1B are an illustration to show an oxygen saturation measuring unit (transmitted light type) mounted in a steering wheel.

FIGS. 2A and 2B are illustrations to show an oxygen saturation measuring unit (reflected light type) mounted in a steering wheel.

FIG. 3 is an illustration to show an embodiment of the invention in an automobile.

FIG. 4 is an illustration to show a method for mounting sensors for controlling the flow of air inside and outside a vehicle cabin.

FIG. 5 is an illustration to show a flow chart of the overall system.

## DETAILED DESCRIPTION OF THE PREFERRED EMBODIMENTS

Hereafter, the preferred embodiments of the invention will be described in detail on the basis of the drawings.

[Embodiment 1]

In this embodiment, an automobile is adopted as a vehicle, but the invention of this application includes not only the automobile but also motor cycle, train, and airplane that are operated by a driver.

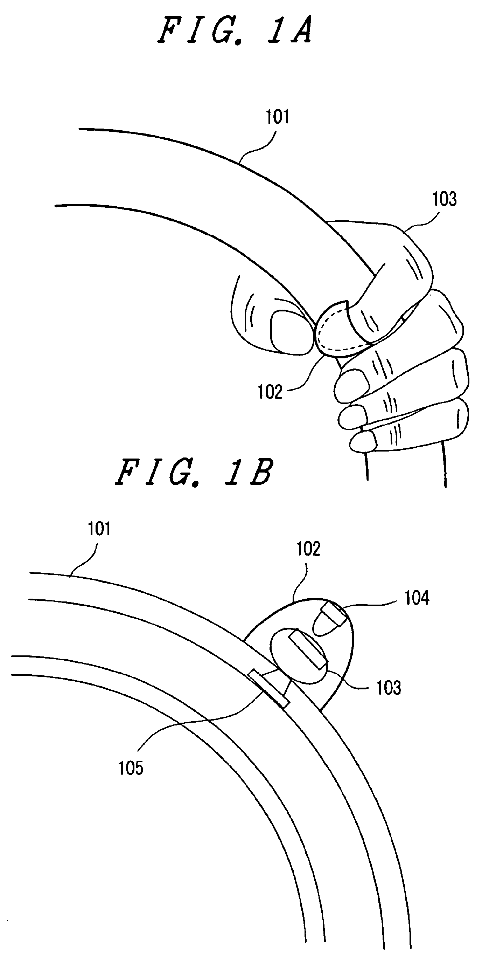

A method for measuring oxygen saturation in the blood of an occupant will be described by use of transmitted light with reference to FIGS. 1A and 1B.

A measuring part 102 is mounted in a steering wheel 101. The measuring part 102 is mounted at a position where a forefinger is placed when the occupant grips the steering wheel 101 by the hand 103. Since the size of hand varies depending on the person, it is effective that a sliding mechanical structure is interposed between the steering wheel 101 and the measuring part 102 to adjust the position of the measuring part 102. A light emitting part 104 and a light receiving part 105 are mounted in the measuring part 102. The light emitting part 104 can be constructed of a light emitting device such as LED and the light receiving part 101 can be constructed of a light receiving device such as photodiode and phototransistor. It is effective to use a device emitting light having a plurality of wavelengths as the light emitting device, but a plurality of devices each emitting light having a single wavelength can be used. In the drawing, electric wirings are omitted. The light emitting part 104 applies light to the hand 103 gripping the steering wheel 101 and the light receiving part 105 receives the light passing through the hand 103.

The intensity of light applied to the finger from the light emitting device and passing through or reflected by the finger varies because the substantial thickness of blood is varied by the pulsation of artery. Pulsating waves can be detected by measuring this variation in the intensity of light. Assuming that the intensity of transmitted light is I, variation in the intensity of transmitted light is $\Delta I$, an extinction coefficient of hemoglobin is $\varepsilon_h$, the concentration of hemoglobin is c, and variation in the thickness of blood is $\Delta D$, a change in extinction $\Delta A$ is approximately expressed as follows by use of a Lambert-Beer's law although the Lambert-Beer's law does not hold for a scatterer like a living body, to be exact.

## [Mathematical formula 1]

$$\Delta A = \lfloor \log I/(I - \Delta I) \rfloor = \varepsilon_h c \cdot \Delta D$$

Assuming that the oxygen saturation of hemoglobin in blood is s, the ratio of existence between oxyhemoglobin and deoxyhemoglobin is s : 1-s and hence assuming that extinction coefficients of oxyhemoglobin and deoxyhemoglobin are $\varepsilon_{oxy}$ and $\varepsilon_{deoxy}$, respectively, an overall extinction coefficient $\varepsilon_h$ of hemoglobin is expressed by the following mathematical formula 2.

## [Mathematical formula 2]

$$\varepsilon_h = s \cdot \varepsilon_{oxy} + (1-s) \cdot \varepsilon_{deoxy}$$

A method for measuring the oxygen saturation of hemoglobin in blood, for example, by selecting two wavelengths will be described. In the case of using two kinds of wavelengths $\lambda_1$, $\lambda_2$ for the light to be emitted, from the mathematical formula 1 and the mathematical formula 2, the following mathematical formula 3 is obtained.

[Mathematical formula 3]

$$R = \Delta A_{\lambda 1}/\Delta A_{\lambda 2} = \lfloor s \cdot \varepsilon_{oxy}(\lambda_1) + (1-s) \cdot \varepsilon_{deoxy}(\lambda_1) \rfloor / \lfloor s \cdot \varepsilon_{oxy}(\lambda_2) + (1-s) \cdot \varepsilon_{deoxy}(\lambda_2) \rfloor$$

With this,

[Mathematical formula 4]

$$s = \lfloor \varepsilon_{deoxy}(\lambda_1) - R \cdot \varepsilon_{deoxy}(\lambda_2) \rfloor / \{ R \lfloor \varepsilon_{oxy}(\lambda_2) - \varepsilon_{deoxy}(\lambda_2) \rfloor - \lfloor \varepsilon_{oxy}(\lambda_1) - R \varepsilon_{deoxy}(\lambda_1) \rfloor \}$$

is derived.

In other words, the oxygen saturation of hemoglobin in blood can be determined by measuring R.

$$R = \Delta A_{\lambda 1}/\Delta A_{\lambda 2}$$

Since R is the ratio of variations in transmitted light in the respective wavelengths, R is actually measured by use of the light receiving device and S is calculated by use of the mathematical formula 4. As to the wavelength, for example, $\lambda_1$ = 660 nm and $\lambda_2$ = 940 nm can be used. It is determined on the basis of the value of R whether or not the supply of oxygen to the vehicle cabin is required.

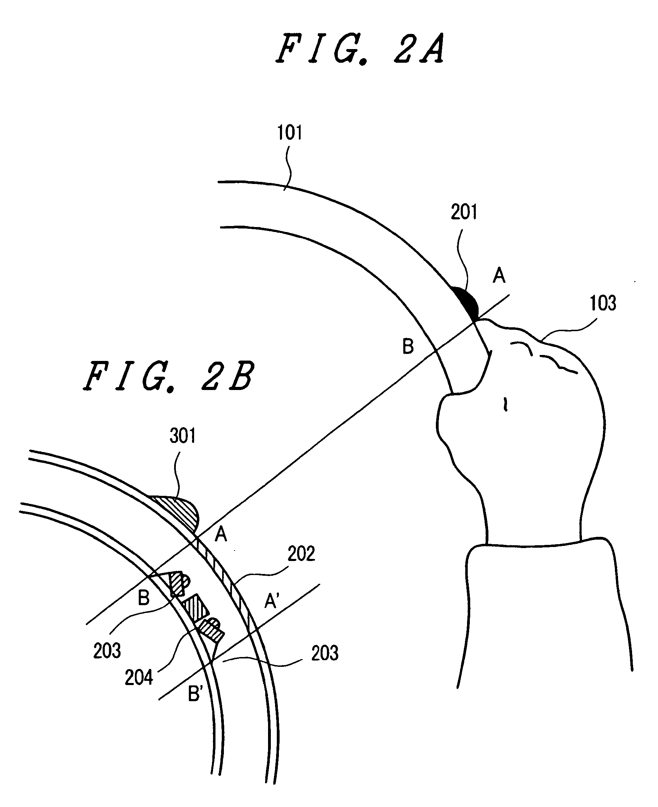

In FIGS. 2A and 2B is shown a method for measuring oxygen saturation in blood by use of reflected light. For example, when the human hand 103 grips the steering wheel 101, a guide 201 for positioning the finger is mounted next to a position where the forefinger is placed. In the steering wheel 101, a measuring window 202 is provided, for example, at a position where the tip of the forefinger is placed. It is desirable that this window 202 is constructed of material capable of transmitted light having the wavelength of a light source. A light emitting device such as LED can be used for the light limiting part 203 and a light receiving part 204 can be constructed of a light receiving device such as photodiode and phototransistor. As to the light emitting part, as shown in FIG. 2B, it is effective that light emitting parts 203 capable of emitting light having different wavelengths are mounted one for each side of the light receiving part 204, or a light emitting part 203 capable of emitting a plurality of wavelengths is mounted on one side of the light receiving part 203. The oxygen saturation is derived from R of the mathematical formula 1, as is the case with the method using transmitted light, and it is determined on the basis of this value whether or not oxygen is to be supplied to the vehicle cabin.

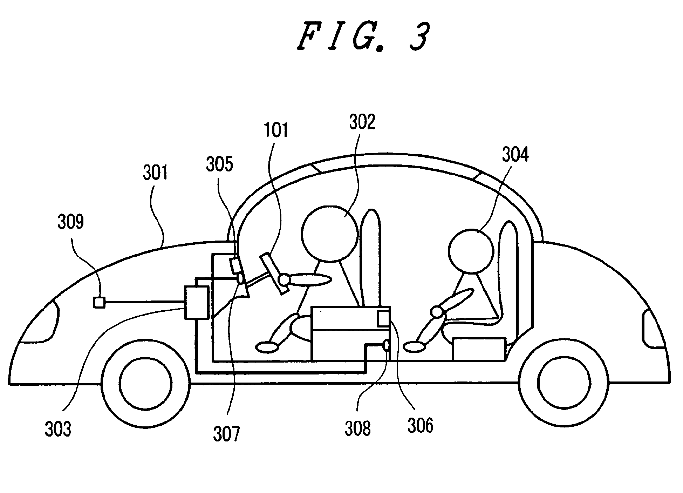

In FIG. 3 is shown an operational mode in an automobile 301. A driver 302 grips the steering wheel 101 and the data of oxygen saturation of the driver obtained in the manner described above is transmitted to a control unit 303 formed of a vehicle-mounted computer or the control unit of an air conditioner (here, electric wirings are omitted). When its value becomes smaller than a predetermined value (for example, 93 %) or continues decreasing monotonously, it is determined that oxygen concentration in the vehicle cabin decreases or that malfunction such as degradation in respiratory function of the occupant occurs. Then, a warning sound or a warning message is issued to the vehicle cabin from a speaker or a warning is issued by the monitoring screen or the sound output of a car navigation system. Further, it is effective that the system is so constructed as to transmit this warning to persons or organizations outside the vehicle by transmitting the malfunction by a mobile phone.

When a fellow passenger 304 is in the vehicle cabin, it is also possible to adopt the following method: the oxygen saturation of the fellow passenger is monitored at the same time and the data of all occupants is compared with each other and when the oxygen saturation of only one occupant is decreased, it is determined that there is a higher probability that the one occupant might be in poor physical condition rather than that oxygen concentration in the vehicle cabin might be decreased to degrade environment and this determination is announced. The oxygen saturation of the fellow passenger can be monitored by providing a sensor like a clothespin of the type in which a finger is pinched by the sensor or a sensor like a finger sack of the type in which a finger is put into the sensor near the seat and by putting the finger into the sensor. A light emitting device and a light receiving device are built in the sensor, as is the case with those mounted in the steering wheel.

The warning triggers the starting of the supply of oxygen. In FIG. 3, the blowoff port 305 of air conditioning for a driver' s seat and the blowoff port 306 of air conditioning for the rear seat are shown as typical examples, but air enriched with oxygen can be blown off from the blowoff ports provided in the ceiling, door, and the like of air conditioner mounted in the vehicle and is not limited to the embodiment shown in the drawing. Here, the piping of air conditioner is not shown and connection to the vehicle-mounted air conditioner will be described later. Further, it is effective to provide a button for starting or stopping the supply of oxygen at one of switches of the air conditioner and to start or stop the supply of oxygen manually.

Further, the state of air in the vehicle cabin, typified by a deficiency of oxygen, can be also detected by methods other than the monitoring of the oxygen saturation of the occupants.

One of the methods is performed by use of an oxygen concentration sensor or a carbon dioxide concentration sensor provided in the vehicle cabin. For example, a galvanic cell type sensor can be used for the oxygen concentration sensor and a solid electrolytic sensor can be used for the carbon dioxide concentration sensor. Oxygen concentration or carbon dioxide concentration is monitored by the oxygen concentration sensor or the carbon dioxide concentration sensor provided near the driver's seat or the oxygen concentration sensor or the carbon dioxide concentration sensor provided in the rear seat and a deficiency of oxygen or an increase in carbon dioxide concentration in the vehicle cabin is announced on the basis of the monitoring result and air enriched with oxygen is blown off from the blowoff ports 305, 306 of the vehicle-mounted air conditioner. Also in this case, the blowoff ports are not limited to these two ports.

Next, a method for preventing the development of high-altitude sickness at the time of driving in highlands by predicting oxygen shortage on the basis of the running information of the vehicle will be described. At As to the altitude information of the vehicle, atmospheric pressure on the ground where the vehicle runs is measured by an atmospheric pressure sensor 309 and altitude is computed on the basis of the atmospheric pressure. The atmospheric pressure sensor is provided also in the vehicle cabin and its position is not limited to the position shown in the drawing. First, a reference altitude is set at a departure point and then a relative altitude measurement of converting a change in atmospheric pressure to an altitude difference is performed. A change in atmospheric pressure can be converted to an altitude difference on the basis of the international standard atmosphere (ISA) data determined by the International Civil Aviation Organization (ICAO). According to the ISA, for example, atmospheric pressure at sea level, 2000 m, and 4000 m are 1013 hPa, 795 hPa, and 616 hPa, respectively, in other words, as the altitude increases by 100 m, the atmospheric pressure decreases by about 12 hPa. Altitude measurement can be easily performed by this method, but it is thought that altitude measurement can be performed more accurately by use of GPS altitude information. Hence, it is effective to use the GPS altitude information, if possible.

In this case, in conjunction with a car navigation system, the rate of increase in altitude when the vehicle runs is determined on the basis of the GPS altitude information obtained from the car navigation system. When the rate of increase in altitude is large, there is probability that the occupant might develop high-altitude sickness, so a warning that it is required to decrease speed or to take a rest is issued to the vehicle cabin. Since the partial pressure of oxygen in the air is decreased at the highlands to decrease the difference in the partial pressure of oxygen between pulmonary alveoli and capillaries to reduce the efficiency of gas exchange in the lung, which might reduce oxygen saturation in the arterial blood to 90 % or less, depending on persons. For this reason, there are cases where symptoms of high-altitude sickness such as headache or nausea appear. It is said that when a person goes from lowlands the altitude above sea level of which is lower than 1500 m or less to highlands the altitude above sea level of which is higher than 2000 m, in particular, 2500 m within a short time of 48 hours or less, or further goes up 500 m per one day from this altitude, the person develops high-altitude sickness in many cases.

Hence, for example, when altitude above sea level at a departure point is 1500morless, it is determined until altitude reaches 2500 m whether or not the rate of increase in altitude is larger than 52 m/hour and after altitude exceeds 2500 m, it is determined whether or not the rate of increase in altitude is larger than 21 m/hour. When a vehicle runs in the lowlands whose altitude above sea level is lower than 2500 m, it is acceptable to issue a light warning of announcing that there is a probability of developing high-altitude sickness to the vehicle cabin, but when the vehicle runs in the highlands whose altitude above sea level is higher than 2500 m, it is effective to issue a warning of reducing speed, lowering altitude, or taking a rest to the vehicle cabin.

The value of oxygen concentration in the atmosphere of the ground where the vehicle runs is computed from the obtained altitude information. This computation is performed, for example, on the basis of the well-known result that the atmospheric pressure varies from 899 hPa to 701 hPa when the altitude varies from 1000 m to 3000 m. Alternatively, it is also effective that an oxygen concentration sensor is provided outside the cabin of a vehicle to measure oxygen concentration actually.

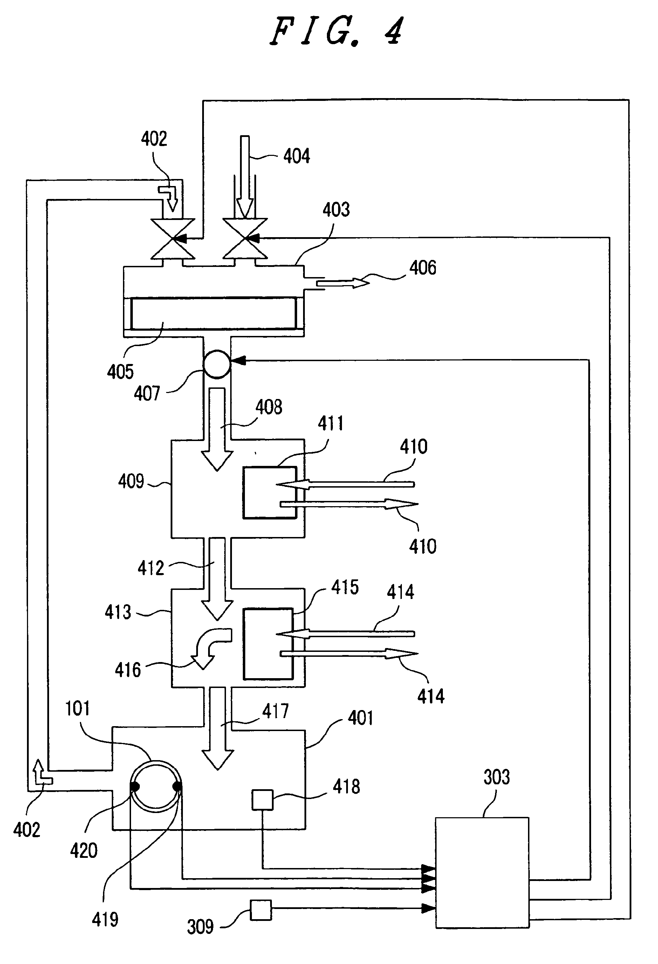

In FIG. 4 is shown an air flow inside and outside a vehicle cabin and a method for mounting sensors for control. As to air inside a vehicle cabin 401, in addition to air coming into or going out of the vehicle cabin through the windows, a portion of air shown by arrow 402 is taken into the blower unit 403 of a vehicle-mounted air conditioner. On the other hand, outside air 404 is also taken into the blower unit 403. An oxygen concentrating part 405 is built in the blower unit 403. Here, a case will be described where a polymeric film type oxygen concentrating part using a material capable of recovering more oxygen by utilizing the fact that a transmission speed is different between oxygen and nitrogen. However, an adsorption type oxygen concentrating part can be also used. Air 406 containing more nitrogen is discharged outside the vehicle. A vacuum pump 407 is mounted on the side where air is taken of the oxygen concentrating part 405 and produces a vacuum, whereby air 408 enriched with oxygen is taken into a cooling unit 409.

Here, since endotherm occurs when a refrigerant 410 is evaporated by an evaporator 411, the air 408 enriched with oxygen is cooled. The air 412 enriched with oxygen and cooled enters a heater unit 413 and is mixed with air 416 warmed by a heater core 415 where engine cooling water 414 is circulated and air 417 having a temperate temperature is blown into the vehicle cabin 401. The control unit 303 processes the information of oxygen saturation sensors 419, 420 mounted in the steering wheel 101, the oxygen concentration sensor or the carbon dioxide sensor 418 arranged in the vehicle cabin, and the atmospheric pressure sensor 309 and controls the valves of suction ports of the inside air 402 that is again taken into the vehicle cabin and the outside air 404, the vacuum pump 407, the cooling unit 409, and the heater unit 413 in a comprehensive manner.

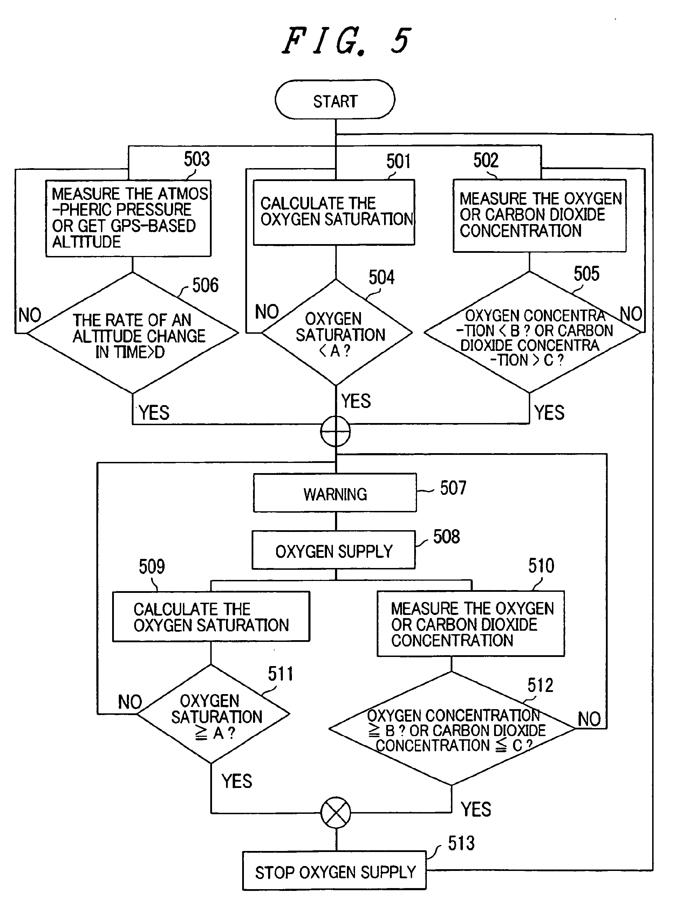

FIG. 5 is a flow chart of the overall system. In order to detect a shortage of oxygen of the occupants, oxygen saturation is measured (501) and in order to detect a shortage of oxygen in the vehicle cabin, oxygen concentration or carbon dioxide concentration is measured (502). It is acceptable that both of the oxygen sensor and the carbon dioxide sensor are mounted but it is also possible to use either of them. The running condition of the vehicle is detected by measuring the atmospheric pressure or by getting GPS altitude information (503). A warning is issued to the vehicle cabin by measuring sensors broadly divided into three kinds of sensors and by determining the results of measurement. The warning can be issued from a speaker in the vehicle cabin or by use of voice or output to the screen in association with a car navigation system. For example, it is effective that when oxygen saturation in blood of an occupant is lower than 96 %, a warning is issued once and that when there is a tendency for the value of oxygen saturation to decrease further, a stronger warning is issued by the voice or screen output. The system is constructed in such a way as to select whose oxygen saturation is monitored.

It is determined whether or not oxygen saturation is not lower than a predetermined value A (504), whether or not oxygen concentration is not lower than a predetermined value B or whether or not carbon dioxide concentration is higher than a predetermined value C (505), and whether or not a rate of the altitude change in time of the position of the moving vehicle is larger than a predetermined value D(506). Then, if any one of them satisfies the condition, the result of determination is notified to the vehicle cabin and a warning is issued (507) . Although a case where only the determination result in the process 504 is YES might occur because of sickness or the like, if only the determination result in the process 505 is YES, it is presumed that the oxygen concentration sensor or the carbon dioxide sensor and the oxygen saturation measuring sensor fail. In this case, a warning is issued to the vehicle cabin by voice announcement or by blinking LED lamps arranged in an instrument panel. Further, although oxygen concentration is lower than 15 % and carbon dioxide concentration reaches 0.1 %, if oxygen saturation is higher than 97 %, it is presumed that the oxygen saturation measuring sensor fails.

Also in this case, a warning is issued to the vehicle cabin by voice announcement or by blinking the LED lamp. Even if any one of the sensors broadly divided into three kinds of sensors fails, the remaining paths in the flow chart can be used. Hence, except when all sensors except for the atmospheric pressure sensor fail, the oxygen concentration can be kept at a predetermined value or more.

If the oxygen concentration sensor and the carbon dioxide concentration sensor fail, the altitude of the ground where the vehicle runs is converted to oxygen concentration on the basis of the measurement result of atmospheric pressure sensor or GPS altitude information and it is determined whether or not the supply of oxygen is required. For example, when altitude varies from 1800 m to 3000 m, oxygen concentration varies from 16.8 % to 14.4 %, so that oxygen concentration can be estimated by the conversion based on this. In order to determine whether or not oxygen in the vehicle cabin is sufficient, it is possible to use a method by which the supply of oxygen is continued until the measurement result of the oxygen saturation sensor reaches 98 %.

The supply of oxygen to the vehicle cabin (508) is performed by using the method shown in FIG. 3. While it is possible to blow oxygen into the vehicle cabin at the same time when a warning is issued, it is also possible to construct a system in such a way as to urge an occupant to check whether or not oxygen is to be blown into the vehicle cabin when a warning is issued and to make the occupant blow off oxygen by his intension.

Further, even if air environment in the vehicle cabin is appropriate, depending on physical conditions of the individual occupants, there is a case where a specific occupant has lower oxygen saturation in the blood. In this case, it is effective to issue a warning that oxygen saturation is lower than a reference value and to supply air containing oxygen having a high concentration of about 30 % or more only from the blowoff port of the air conditioner near the occupant. In this case, it is also possible to mount an oxygen cylinder in the vehicle and to supply oxygen from the cylinder. With the above-described control, it is possible to condition air environment in the vehicle cabin according to the physical conditions of the individual occupants.

When the supply of oxygen is started (508), the oxygen saturation in the blood of the occupant is measured in succession (509), whereas oxygen concentration or carbon dioxide concentration in the vehicle cabin is measured (510). Then, it is determined (511) whether or not the oxygen saturation reaches the predetermined value A, it is determined (512) whether or not the oxygen concentration reaches the predetermined value B or whether or not the carbon dioxide concentration reaches the predetermined value C. When both determination results are YES, the supply of oxygen is stopped (513). Here, it is effective that the predetermined values A, B, and C, as guides, are set, for example, at 98 %, 21 %, and 0.06 %.

While the above embodiment has been described on the assumption that the invention is applied to an automobile, the invention can be applied to vehicles such as airplane and train. Further, the invention relates to environment conditioning in a place where humans exist and can be used in houses, buildings, and underground construction sites and has a wide application area.

**Claims**

1. A control unit controlling a ratio of constituents of air supplied to a vehicle cabin (401) by an air conditioner **characterized in that** said ratio of constituents of air is controlled according to oxygen saturation in blood of a driver or a fellow passenger,
   which is detected by an oxygen saturation detecting part (102, 418).

2. Control unit according to claim 1,
   **characterized in that** when oxygen saturation detected by the oxygen saturation detecting part (102, 418) is lower than a predetermined threshold, the air conditioner supplies air enriched with oxygen to the vehicle cabin (401).

3. Control unit according to claim 1 or 2,
   **characterized in that** when oxygen concentration in the vehicle cabin (401), which is detected by an oxygen concentration detecting part (102, 418), is detected to be lower than a predetermined threshold, the air conditioner supplies air enriched with oxygen to the vehicle cabin (401).

4. Control unit according to at least one of the preceding claims,
   **characterized in that** when carbon dioxide concentration in the vehicle cabin (401), which is detected by a carbon dioxide concentration detecting part (418), is detected to be higher than a predetermined threshold, the air conditioner supplies air enriched with oxygen to the vehicle cabin (401).

5. Control unit according to at least one of the preceding claims,
   **characterized in that** when a rate of the altitude change in time of the position of the moving vehicle, which is detected by an altitude detecting part, is detected to be higher than a predetermined threshold, the air conditioner supplies air enriched with oxygen to the vehicle cabin (401) .

6. A control unit according to any of the preceding claims, **characterized in that** the control unit makes an air conditioner supply air enriched with oxygen to a vehicle cabin (401) when carbon dioxide concentration in the vehicle cabin (401), which is detected by a carbon dioxide concentration detecting part (418), is detected to be higher than a predetermined threshold.

7. A control unit that makes an air conditioner supply air enriched with oxygen to the vehicle cabin (401) **characterized in that** said air is supplied to the vehicle cabin when a rate of the altitude change in time of the position of the moving vehicle, which is detected by an altitude detecting part, is detected to be higher than a predetermined threshold.

8. Control unit according to at least one of the preceding claims,
   wherein a warning is outputted in dependence on a value of the saturation in blood.

9. An air conditioning system comprising a control unit according to any of the preceding claims.

10. A method for conditioning air, comprising the steps of:

   detecting oxygen saturation in blood of a driver or a fellow passenger (304) and
   controlling oxygen concentration of air supplied to a vehicle cabin from an air conditioner according to the detected oxygen saturation.

**Patentansprüche**

1. Steuerungseinheit, die ein Bestandteilsverhältnis von Luft, die einem Kraftfahrzeuginnenraum (401) durch eine Klimaanlage zugeführt wird, steuert, **dadurch gekennzeichnet, dass** das Luftbestandteilsverhältnis nach Maßgabe der Sauerstoffsättigung im Blut eines Fahrers oder Beifahrers gesteuert wird, die durch ein Sauerstoffsättigungserfassungsteil (102, 418) erfasst wird.

2. Steuerungseinheit nach Anspruch 1, **dadurch gekennzeichnet, dass**, wenn die durch das Sauerstoffsättigungserfassungsteil (102, 418) erfasste Sauerstoffsättigung niedriger als ein vorgegebener Schwellwert ist, die Klimaanlage dem Kraftfahrzeuginnenraum (401) mit Sauerstoff angereicherte Luft zuführt.

3. Steuerungseinheit nach Anspruch 1 oder 2, **dadurch gekennzeichnet, dass**, wenn erfasst wird, dass die Sauerstoffkonzentration im Kraftfahrzeuginnenraum (401), die durch ein Sauerstoffkonzentrationserfassungsteil (102, 418) erfasst wird, niedriger als ein vorgegebener Schwellwert ist, die Klimaanlage dem Kraftfahrzeuginnenraum (401) mit Sauerstoff angereicherte Luft zuführt.

4. Steuerungseinheit nach mindestens einem der vorhergehenden Ansprüche, **dadurch gekennzeichnet, dass**, wenn erfasst wird, dass die Kohlendioxidkonzentration im Kraftfahrzeuginnenraum (401), die durch ein Kohlendioxidkonzentrationserfassungsteil (418) erfasst wird, höher als ein vorgegebener Schwellwert ist, die Klimaanlage wird, höher als ein vorgegebener Schwellwert ist, die Klimaanlage dem Kraftfahrzeuginnenraum (401) mit Sauerstoff angereicherte Luft zuführt.

5. Steuerungseinheit nach mindestens einem der vorhergehenden Ansprüche, **dadurch gekennzeichnet, dass**, wenn erfasst wird, dass die Höhenänderungsrate im Lauf der Zeit der Position des sich bewegenden Fahrzeugs, die durch ein Höhenerfassungsteil erfasst wird, höher als ein vorgegebener Schwellwert ist, die Klimaanlage dem Kraftfahrzeuginnenraum (401) mit Sauerstoff angereicherte Luft zuführt.

6. Steuerungseinheit nach irgendeinem der vorhergehenden Ansprüche, **dadurch gekennzeichnet, dass** die Steuerungseinheit die Klimaanlage veranlasst, einem Kraftfahrzeuginnenraum (401) Luft zuzuführen, die mit Sauerstoff angereichert ist, wenn erfasst wird, dass die Kohlendioxidkonzentration im Kraftfahrzeuginnenraum (401), die durch ein Kohlendioxidkonzentrationserfassungsteil (418) erfasst wird, höher als ein vorgegebener Schwellwert ist.

7. Steuerungseinheit, die eine Klimaanlage veranlasst, dem Kraftfahrzeuginnenraum (401) mit Sauerstoff angereicherte Luft zuzuführen, **dadurch gekennzeichnet, dass** die Luft dem Kraftfahrzeuginnenraum zugeführt wird, wenn erfasst wird, dass die Höhenänderungsrate im Lauf der Zeit der Position des sich bewegenden Fahrzeugs höher als ein vorgegebener Schwellwert ist.

8. Steuerungseinheit nach mindestens einem der vorhergehenden Ansprüche, wobei eine Warnung in Abhängigkeit von einem Sättigungswert im Blut ausgegeben wird.

9. Klimaanlagesystem mit einer Steuerungseinheit nach irgendeinem der vorhergehenden Ansprüche.

10. Verfahren zur Luftklimatisierung mit folgenden Schritten:

    Erfassen der Sauerstoffsättigung im Blut eines Fahrers oder Beifahrers (304) und
    Steuern der Sauerstoffkonzentration der Luft, die einem Kraftfahrzeuginnenraum von einer Klimaanlage nach Maßgabe der erfassten Sauerstoffsättigung zugeführt wird.

**Revendications**

1. Unité de régulation régulant un rapport des constituants de l'air fourni à une cabine de véhicule (401) par un climatiseur, **caractérisée en ce que** ledit rapport des constituants de l'air est régulé en fonction de la saturation en oxygène dans le sang d'un conducteur ou d'un passager, qui est détectée par une partie détection de la saturation en oxygène (102, 418).

**2.** Unité de régulation selon la revendication 1,
**caractérisée en ce que**, quand la saturation en oxygène détectée par la partie détection de la saturation en oxygène (102, 418) est inférieure à un seuil prédé fini, le climatiseur fournit de l'air enrichi en oxygène à la cabine de véhicule (401).

**3.** Unité de régulation selon la revendication 1 ou 2,
**caractérisée en ce que**, quand la concentration en oxygène dans la cabine du véhicule (401), qui est détectée par une partie détection de la concentration en oxygène (102, 418), est détectée comme étant inférieure à un seuil prédéfini, le climatiseur fournit de l'air enrichi en oxygène à la cabine de véhicule (401).

**4.** Unité de régulation selon au moins l'une des revendications précédentes,
**caractérisée en ce que**, quand la concentration en dioxyde de carbone dans la cabine du véhicule (401), qui est détectée par une partie détection de la concentration en dioxyde de carbone (418), est détectée comme étant supérieure à un seuil prédéfini, le climatiseur fournit de l'air enrichi en oxygène à la cabine de véhicule (401).

**5.** Unité de régulation selon au moins l'une des revendications précédentes,
**caractérisée en ce que**, quand un niveau de changement d'altitude au cours du déplacement du véhicule en mouvement, qui est détectée par une partie détection de l'altitude, est détecté comme étant supérieur à un seuil prédéfini, le climatiseur fournit de l'air enrichi en oxygène à la cabine de véhicule (401).

**6.** Unité de régulation selon l'une quelconque des revendications précédentes,
**caractérisée en ce que** l'unité de régulation fait fournir par le climatiseur de l'air enrichi en oxygène à une cabine de véhicule (401), quand la concentration en dioxyde de carbone dans la cabine du véhicule (401), qui est détectée par une partie détection de la concentration en dioxyde de carbone (418), est détectée comme étant supérieure à un seuil prédéfini.

**7.** Unité de régulation qui fait fournir par un climatiseur de l'air enrichi en oxygène à la cabine de véhicule (40 1),
**caractérisée en ce que** ledit air est fourni à la cabine de véhicule quand un niveau de changement d'altitude au cours du déplacement du véhicule en mouvement, qui est détectée par une partie détection de l'altitude, est détecté comme étant supérieur à un seuil prédéfini.

**8.** Unité de régulation selon au moins l'une des revendications précédentes,
dans laquelle un avertissement est émis en fonction d'une valeur de saturation dans le sang.

**9.** Système de climatisation comprenant une unité de régulation selon l'une quelconque des revendications précédentes.

**10.** Procédé de climatisation, comprenant les étapes de :

    détection de la saturation en oxygène dans le sang d'un conducteur ou d'un passager (304) et
    régulation de la concentration en oxygène de l'air fourni à une cabine de véhicule depuis un climatiseur selon la saturation en oxygène détectée.

# FIG. 1A

101

103

102

# FIG. 1B

101

102

104

103

105

# FIG. 2A

# FIG. 2B

# *FIG. 3*

# FIG. 4

# FIG. 5

START

503 — MEASURE THE ATMOS-PHERIC PRESSURE OR GET GPS-BASED ALTITUDE

501 — CALCULATE THE OXYGEN SATURATION

502 — MEASURE THE OXYGEN OR CARBON DIOXIDE CONCENTRATION

506 — THE RATE OF AN ALTITUDE CHANGE IN TIME>D    NO    YES

504 — OXYGEN SATURATION < A ?    NO    YES

505 — OXYGEN CONCENTRA-TION < B ? OR CARBON DIOXIDE CONCENTRA-TION > C ?    NO    YES

507 — WARNING

508 — OXYGEN SUPPLY

509 — CALCULATE THE OXYGEN SATURATION

510 — MEASURE THE OXYGEN OR CARBON DIOXIDE CONCENTRATION

511 — OXYGEN SATURATION ≧ A ?    NO    YES

512 — OXYGEN CONCENTRATION ≧ B ? OR CARBON DIOXIDE CONCENTRATION ≦ C ?    NO    YES

513 — STOP OXYGEN SUPPLY